# EUROPEAN PATENT SPECIFICATION

(11) **EP 3 698 138 B1**
(45) Date of publication and mention of the grant of the patent: **22.11.2023**
(21) Application number: 18797151.0
(22) Date of filing: 19.10.2018
(51) Int. Cl.: G01N 33/564, G01N 33/574, C07K 14/47

(54) **ASSAY AND KIT FOR THE DIAGNOSIS OF OVARIAN CARCINOMA**
TEST UND KIT ZUR DIAGNOSE VON EIERSTOCKKARZINOMEN
ESSAI ET KIT POUR LE DIAGNOSTIC DU CANCER DE L'OVAIRE

(30) Priority: 18.10.2017 IT 201700117860
(43) Date of publication of application: 26.08.2020
(73) Proprietor: Molipharma R&D S.r.l., 86100 Campobasso (CB) (IT)
(72) Inventor: SCAMBIA, Giovanni, 86100 Campobasso CB (IT); GALLO, Daniela, 86100 Campobasso CB (IT); PETRILLO, Marco, 86100 Campobasso CB (IT); MARTINELLI, Enrica, 86100 Campobasso (CB) (IT); BATTAGLIA, Alessandra, 00168 Roma (IT)
(74) Representative: Predazzi, Valentina
(86) International application number: PCT/IB2018/058160
(87) International publication number: WO 2019/077579

(56) References cited:
- WO-A1-2007/046796
- WO-A1-2017/178948
- WO-A2-2006/076100
- MASAHIRO MUKAI ET AL: "Recombinant Mammalian Tubulin Polyglutamylase TTLL7 Performs both Initiation and Elongation of Polyglutamylation on [beta]-Tubulin through a Random Sequential Pathway +", BIOCHEMISTRY, vol. 48, no. 5, 10 February 2009 (2009-02-10), pages 1084-1093, XP055467115, US ISSN: 0006-2960, DOI: 10.1021/bi802047y
- DRABEROVA E ET AL: "EXPRESSION OF CLASS III BETA-TUBULIN IN NORMAL AND NEOPLASTIC HUMAN TISSUES", HISTOCHEMISTRY AND CELL BIOLOGY, SPRINGER, BERLIN, DE, vol. 109, no. 3, 1 March 1998 (1998-03-01) , pages 231-239, XP001055654, ISSN: 0948-6143, DOI: 10.1007/S004180050222
- M. A. LOPATA: "In vivo microtubules are copolymers of available beta-tubulin isotypes: localization of each of six vertebrate beta-tubulin isotypes using polyclonal antibodies elicited by synthetic peptide antigens", THE JOURNAL OF CELL BIOLOGY : JCB, vol. 105, no. 4, 1 October 1987 (1987-10-01), pages 1707-1720, XP055467112, US ISSN: 0021-9525, DOI: 10.1083/jcb.105.4.1707
- G. FERRANDINA: "Class III beta-Tubulin Overexpression Is a Marker of Poor Clinical Outcome in Advanced Ovarian Cancer Patients", CLINICAL CANCER RESEARCH, vol. 12, no. 9, 1 May 2006 (2006-05-01), pages 2774-2779, XP055320558, US ISSN: 1078-0432, DOI: 10.1158/1078-0432.CCR-05-2715

## Description

### DESCRIPTION

The present invention relates to the identification of markers with high sensitivity and specificity for the diagnosis and the prognosis of ovarian carcinoma, diagnostic or prognostic methods comprising the use of such markers, and diagnostic or prognostic kits comprising such markers.

### PRIOR ART

Ovarian carcinoma is the first cause of death among gynaecologic tumours. Owing to the high growth rate and invasive capacity of ovarian carcinoma, most patients present an advanced-stage disease at diagnosis. Moreover, despite cytoreductive surgery progresses and the introduction of carboplatin/paclitaxel combinations as a conventional therapeutic regimen, intrinsic or acquired drug resistance remains the main determinant of chemotherapy failure and, as a result, of a fatal clinical outcome. The best strategy for an effective improvement of the prognosis is represented by an early diagnosis of disease that can be carried out through use of clinically validated biomarkers.

Microtubules are intracellular structures important for many cellular processes, such as mitosis, motility and intracellular transportation. They are comprised of alpha- and beta-tubulin monomers, which spontaneously assemble to form heterodimers. Class III tubulin (TUBB3), encoded by the tubb3 gene, is a protein constitutively expressed in human neuronal cells in the central and peripheral nervous system and in Sertoli cells of the seminiferous tubules of the testis (Mariani et al., 2015). This protein is not normally expressed in epithelial cells, whereas it is overexpressed in many types of carcinoma, such as the lung, stomach, prostatic and ovarian ones (Mariani et al., 2015; Terry et al., 2009; Yang et al., 2014; Hwang et al.,2013; Ferrandina et al., 2006). Recent studies demonstrated how, although TUBB3 overexpression in many tumours, among which ovarian tumour, is associated with unfavourable prognosis (Mozzetti et al., 2005; Ferrandina et al., 2006; Raspaglio et al., 2008 Mariani et al., 2015; Izutsu et al., 2008; Kavallaris, 2010; Roque et al., 2014), there is no correlation between TUBB3 overexpression and aggressiveness in other tumours, such as cervical ones (Ferrandina et al., 2007). Finally, TUBB3 role remains controversial in other neoplastic pathologies, such as, e.g., breast carcinoma, in which hormonal influence plays a key role in TUBB3 expression(Mariani et al., 2015).

However, with regard to ovarian carcinoma, it emerged how, given the inherent heterogeneity of the disease, immunohistochemistry is not a suitable technique for a correct quantitation of TUBB3 expression, as TUBB3 levels can be sensibly different in different sections of the same tumour. Moreover, the immunohistochemistry method is expensive, and the histopathological assessment of expression is by its own nature subjective, providing in the last analysis categorical, non-continuous data. In fact, the assessment of TUBB3 expression using immunohistochemistry as a method requires:
- an invasive intervention for bioptic sample collection;
- the intervention of several professionals, such as technicians, biologists specialized in histopathology, and pathologists qualified and expert in the tissue to be studied;
- a relevant commitment in terms of time, as the various biopsies to be studied are first fixed, then processed and finally embedded, before being used for analysis;
- a high economical commitment, given the number of highly specialized persons involved and the materials consumed.

All this limited TUBB3 use as biomarker of diagnosis and/or prognosis in ovarian tumour.

In the initial stage of cancer development and during its progression, mutations are produced which lead to protein overexpression, or to expression of ectopic proteins that, when recognized by the immune system, can induce production of specific antibodies. In fact, in the last years many studies demonstrated the presence of autoantibodies against tumour-associated antigens (TAA) in the blood of cancer patients. These tumour-specific autoantibodies, also thanks to their biochemical characteristics of stability and high concentration in serum, can easily be identified and, therefore, can be considered reliable biomarkers in the early diagnosis of a tumour, such as oesophageal, lung, ovarian, colorectal carcinomas and melanoma (Defresne F, Bouzin C, Guilbaud C, Dieu M, Delaive E, Michiels C,et al. Differential influence of anticancer treatment and angiogenesis on the seric titer of autoantibody used as tumor and metastasis biomarker. Neoplasia; 2010.vol12 July, 565-570; Grassadonia A, Tinari N, Natoli C, Yahalom G, lacobelli S. Circulating auto-antibodies to LGALS3BP: a novel biomarker for cancer. Dis Markers. 2013;35(6):747-52). Moreover, in numerous studies it has been demonstrated that the presence of autoantibodies specific against TAA is associated with a greater probability of recurrence; therefore, the dosage of anti-TAA autoantibodies can constitute a valid support in laboratory diagnosis associated with assessment of tumour aggressiveness.

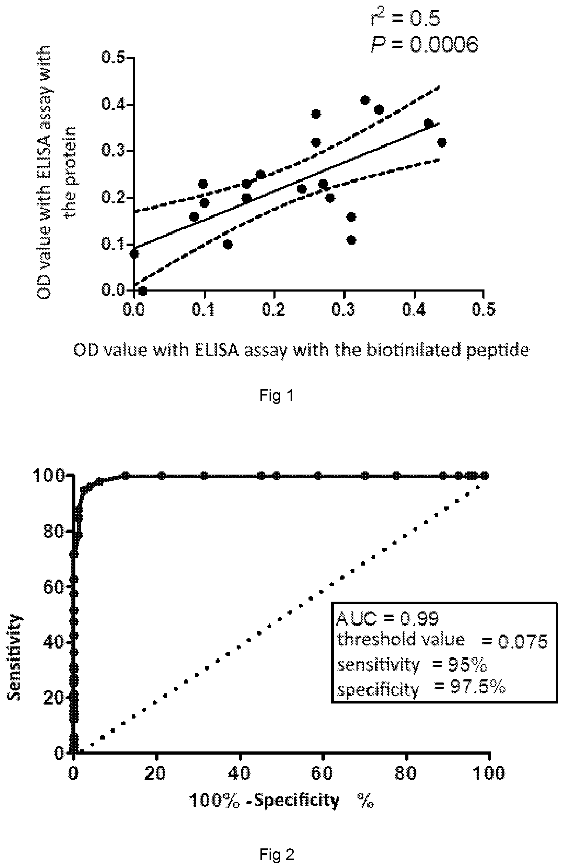

Bansal D et al, in 2009 (Bansal D, Herbert F, Lim P, Deshpande P, Bécavin C, Guiyedi V, et al. IgG autoantibody to brain beta tubulin III associated with cytokine cluster-II discriminate cerebral malaria in central India. PLoS One. 2009 Dec 14;4(12):e8245.) demonstrated (by ELISA, enzyme-linked immunosorbent assay, method) the presence of IgG-class anti-TUBB3 autoantibodies in patients affected by cerebral malaria, as a consequence of neuronal damage induced by *Plasmodium falciparum* infection (Bansal et al., 2009). In this work, the authors do not specify the production and purification characteristics of the TUBB3 recombinant protein utilized in the ELISA assay. By analogy, the authors of the invention assumed that in ovarian tumour, after intracellular component release in tumour microenvironment following cell death, TUBB3 may become immunogenic, thereby inducing the production of autoantibodies detectable in patients' sera, in analogy to numerous other autoantibodies against tumour-associated antibodies (TAA) (Reuschenbach et al., 2009). In this connection, a research previously carried out by the Authors of the invention demonstrated the presence of anti-TUBB3 autoantibodies in the serum of patients with ovarian carcinoma, and the correlation between the concentration of these antibodies and the disease diagnosis and prognosis. For the dosage of serum levels of anti-TUBB3 autoantibodies an ELISA (enzyme-linked immunosorbent assay) assay was set up, using the entire human TUBB3 recombinant protein produced in insect cells as antigen. Data obtained from said research were the subject of a patent (Patent application N° 102016000037019; Application Number: PCT/IB2017/052048). As it may be inferred from Figures 1 and 2, published in PCT/IB2017/052048, the concentration of anti-TUBB3 autoantibodies has prognostic value as regards disease-free time (Fig.1) and overall survival (Fig. 2). Figure 1, published in PCT/IB2017/052048, shows the results of a Kaplan Meier analysis of progression-free survival (PFS) in patients affected by ovarian carcinoma. PFS curves in patients with ovarian carcinoma depending on the concentration of anti-TUBB3 autoantibodies (in the present description, and in the figures, also referred to as auto-AbTUBB3). Continuous line: low levels of anti-TUBB3 autoantibodies; broken line: high levels of anti-TUBB3 autoantibodies. Figure 2, published in PCT/IB2017/052048, shows a Kaplan-Meier analysis of overall survival (OS) in patients affected by ovarian carcinoma. OS curves in patients with ovarian carcinoma depending on the concentration of anti-TUBB3 autoantibodies (in the present description, and in the figures, also referred to as auto-AbTUBB3). Continuous line: low levels of anti-TUBB3 autoantibodies; broken line: high levels of anti-TUBB3 autoantibodies.

The above-indicated Patent Applications disclose assays wherein at a cut-off value of 0.15 as cut-off between levels of anti-TUBB3 antibodies in healthy subjects (A₄₅₀ <0.15) and in diseased subjects (A₄₅₀ >0.15), a calculated 90% sensitivity and 95.5% specificity percentage is had.The patent application WO2006/076100A2 discloses peptides from the C-terminus of beta-tubulin III as tumoral markers useful for identifying a patient with cancer for treatment with a chemical compound.

Similarly, Draberova E. et al. ("Expression of class III beta-tubulin in normal and neoplasic human tissues", Histochem Cell Biol. 1998) disclose the use of monoclonal antibodies against peptides from the C-terminus of the human class III beta tubulin isotype as tumoral markers as well as for immunohistochemical characterization of tumors of neuronal origin.

Also, Lopata M.A. et al. ("In vivo microtubules are copolymers of available beta-tubulin isotypes: localization of each of six vertebrate beta-tubulin isotypes using polyclonal antibodies elicited by synthetic peptide antigens", J Cell Biol. 1987) disclose antibodies against peptides from the C-terminus of class III beta tubulin.

On the other hand, the patent application WO2007/046796A1 describes isolated polypeptides derived from the C-terminus of a tubulin isoform for use in methods of treatment of cell proliferative disorders.

Mukai et al. ("Recombinant mammalian tubulin polyglutamylase TTLL7 performs both initiation and elongation of polyglutamylation on beta-tubulin through a random sequential pathway", Biochemistry 2009) investigated the polyglutamylation reaction performed by the beta-tubulin-selective polyglutamilase TTLL7 by employing a recombinant enzyme and *in vitro* reaction.

Given ovarian carcinoma frequency and seriousness, any improvement in the diagnosis or prognosis thereof is desirable.

An ELISA kit for the dosage of IgM-class autoantibodies specific for TUBB3 is currently on the market.

### SUMMARY OF THE INVENTION

Surprisingly, The Authors of the present invention have discovered that the use of an antigen different from the whole TUBB3 protein for the detection of anti-TUBB3 antibodies enables to obtain a concomitant increase of sensitivity and specificity (the increase of one of the two values normally corresponds to a decrease of the other one, threshold values being equal), greater than that reported in PCT/IB2017/052048, at threshold values lower than those reported in PCT/IB2017/052048.

Compared to the system reported in PCT/IB2017/052048, the Authors of the present invention have therefore carried out modification that determined an important improvement of the sensitivity and specificity of the assay in terms of diagnostic accuracy. Moreover, the novel procedure allows the development of a diagnostic kit optimized for a maximum ease of use and a remarkable reduction of costs.

In particular, the dosage of anti-TUBB3 autoantibodies according to the present invention could be carried out in all analysis laboratories, as simple, quick and inexpensive. The methodology does not require a complex pre-analytical stage of the sample to be analysed and uses a few microliters of serum and enables the development of a method and of a kit optimized for the utmost ease of use for early diagnosis of ovarian tumour. The Authors of the present invention discovered that, by carrying out the detection of antiTUBB3 autoantibodies in serum as described in PCT/IB2017/052048 (see, e.g., claims 1-3) by ELISA assay, using a peptide having a sequence corresponding to the last from 16 to 45 C-terminal amino acids of the TUBB3 protein, it is possible to halve the cut-off identified in PCT/IB2017/052048 and increase, surprisingly, both sensitivity and specificity of the results obtained.

The Inventors therefore set up a diagnostic and/or prognostic method, based on the detection of these autoantibodies in patients' sera, which leads to significantly improving the already effective method disclosed in PCT/IB2017/052048.

It is therefore object of the invention the subject matter as defined in the appended claims.

Therefore, the invention relates to:
- an *in vitro* method for the diagnosis of ovarian carcinoma in a patient, comprising the following steps:
   a) determining the concentration C of anti-TUBB3 autoantibodies in a serum sample of said patient;
   b) comparing said concentration C of autoantibodies to a threshold value x, wherein a concentration of said autoantibodies higher than said threshold value identifies the presence of ovarian carcinoma in said patient
   wherein said determining in step a) is carried out by ELISA assay with detection of said antibodies with a peptide having a sequence selected from SEQ ID NO 1 or SEQ ID NO 3 or SEQ ID NO 4;
- an *in vitro* method for the diagnosis of ovarian carcinoma and the prognosis of survival free from progression and/or of overall survival expectancy in case of presence of said ovarian carcinoma in a patient, comprising the following steps:
   a) determining the concentration C of anti-TUBB3 autoantibodies in a serum sample of said patient;
   b) comparing said concentration C of autoantibodies to a threshold value x, wherein a concentration of said autoantibodies higher than said threshold value x identifies the presence of ovarian carcinoma in said patient; and
   c) when said patient is positive to ovarian carcinoma, comparing said concentration C of autoantibodies to a threshold value y; wherein for C>y an increasing difference between said concentration C and said second threshold value y corresponds to a decreasing of survival free from progression and/or overall survival expectancy of said patient and for C<y an increasing difference between said concentration C and said threshold value y corresponds to an increasing of survival free from progression and/or overall survival expectancy of said patient.
   wherein said determining in step a) is carried out by ELISA assay with detection of said antibodies with a peptide having a sequence selected from SEQ ID NO 1 or SEQ ID NO 3 or SEQ ID NO 4;
- an *in vitro* method for the prognosis of survival free from progression and/or of overall survival expectancy in a patient affected by ovarian carcinoma, comprising the following steps:
   a) determining the concentration C of anti-TUBB3 autoantibodies in a serum sample of said patient;
   c) comparing said concentration C of autoantibodies to a threshold value y;
   wherein for C>y an increasing difference between said concentration C and said second threshold value y corresponds to a decreasing of survival free from progression and/or overall survival expectancy of said patient and for C<*y* an increasing difference between said concentration C and said threshold value y corresponds to an increasing of survival free from progression and/or overall survival expectancy of said patient. wherein said determining in step a) is carried out by ELISA assay with detection of said antibodies with a peptide having a sequence selected from SEQ ID NO 1 or SEQ ID NO 3 or SEQ ID NO 4;
- peptides suitable for carrying out said methods,
- kits containing reagents suitable for carrying out said methods, including the peptideas defined in the appended set of claims.

### DETAILED DESCRIPTION OF THE FIGURES

Figure 1. Correlation analysis between Optical Density (OD) values obtained with the ELISA methodology, using the whole protein (related to the preceding patent) and the method of the invention (biotinylated peptide having SEQ ID NO 1 - streptavidin-coated) for the dosage of anti-TUBB3 autoantibodies levels (n=20 patients).
Figure 2. ROC curve related to serum concentration of anti-TUBB3 autoantibodies.
Figure 3. Intra-assay variability determined on 5 replicates of serum samples with decreasing auto-TUBB3 concentrations. CV: variation coefficient.
Figure 4. Inter-assay variability determined on 3 replicates of serum samples with decreasing auto-TUBB3 concentrations. CV: variation coefficient.

### DETAILED DESCRIPTION OF THE SEQUENCES

SEQ ID NO 1: Peptide containing the last 27 amino acids (aa) specific for the C-terminal region of human protein TUBB3 (corresponding to aa in position 424-450 of SEQ ID NO 2).
   QYQDATAEEEGEMYEDDEEESEAQGPK
SEQ ID NO 2 sequence of human TUBB3 isoform 1 (NP_006077.2).
SEQ ID NO 3: Peptide containing the last 28 amino acids specific for the C-terminal region of human protein TUBB3 (corresponding to aa in position 423-450 of SEQ ID NO 2).
   QQYQDATAEEEGEMYEDDEEESEAQGPK
SEQ ID NO 4: Peptide containing the last 26 amino acids specific for the C-terminal region of human protein TUBB3 (corresponding to aa in position 425-450 of SEQ ID NO 2).
   YQDATAEEEGEMYEDDEEESEAQGPK

### GLOSSARY

TUBB3 or human tubulin beta 3 chain.

The term TUBB3 represents human protein tubulin beta 3 or tubulin beta III, well-known in the literature.

TUBB3 protein is known in the literature, as well as numerous variants thereof. Known variants exhibit amino acid variations with respect to isoform 1 reported in SEQ ID NO 2 reported in Table 1 below.

The transcript corresponding to tubulin beta 3 isoform 1 is NM_006086.3 (Reference sequence obtained from National Center for Biotechnology Information, NCBI, databank).

**Table 1**

| **Name** | **Position** | **Variation** |
|---|---|---|
| Natural variantⁱVAR_062758 | 62 | R → Q |
| Natural variantⁱVAR_062759 | 262 | R → C |
| Natural variantⁱVAR_062760 | 262 | R → H |
| Natural variantⁱVAR_062761 | 302 | A → T |
| Natural variantⁱVAR_062762 | 380 | R → C |
| Natural variantⁱVAR_062763 | 410 | E → K |
| Natural variantⁱVAR_062764 | 417 | D → H |
| Natural variantⁱVAR_062765 | 417 | D → N. |
| Natural variantⁱVAR_066206 | 178 | T→ M. |
| Natural variantⁱVAR_066207 | 205 | E → K |
| Natural variantⁱVAR_066208 | 302 | A → V |
| Natural variantⁱVAR_066209 | 323 | M → V |

For the purposes of the invention, the term TUBB3 or Tubulin 3 denotes SEQ ID NO 2 and all the natural variants thereof.

For the purposes of the present invention, the term "patients affected by benign gynaecological pathologies", or "subjects affected by benign gynaecological pathologies" means patients affected by benign uterus or ovarian tumours.

### DETAILED DESCRIPTION OF THE INVENTION

As already described above, the present invention provides new diagnostic and/or prognostic methods, further improved with respect to the prior art in the field of oncological diagnostics, that are carried out on serum samples.

The methods of the invention enable a rapid and very reliable diagnosis about the presence or absence of ovarian carcinoma, and in a non-invasive manner, since a sample of peripheral blood from the patient suffices to perform the dosage (assay). Moreover, the methods of the present invention enable to acquire in very quick times information of remarkable relevance for patients affected by ovarian carcinoma, about the aggressiveness of the same carcinoma, with a sensitivity and a specificity remarkably higher than what is described in the prior art.

An object of the present invention is represented by an *in vitro* method for the diagnosis of ovarian carcinoma in a patient, comprising the following steps:
a) determining the concentration C of anti-TUBB3 autoantibodies in a serum sample of said patient;
b) comparing said concentration C of autoantibodies to a threshold value x, wherein a concentration of said autoantibodies higher than said threshold value identifies the presence of ovarian carcinoma in said patient
wherein said determining in step a) is carried out by ELISA assay with detection of said antibodies with a peptide having a sequence selected from SEQ ID NO 1 or SEQ ID NO 3 or SEQ ID NO 4.

Another object of the invention is an *in vitro* method for the diagnosis of ovarian carcinoma and the prognosis of survival free from progression and/or of overall survival expectancy in case of presence of said ovarian carcinoma in a patient, comprising the following steps:
a) determining the concentration C of anti-TUBB3 autoantibodies in a serum sample of said patient;
b) comparing said concentration C of autoantibodies to a threshold value x, wherein a concentration of said autoantibodies higher than said threshold value x identifies the presence of ovarian carcinoma in said patient; and
c) when said patient is positive to ovarian carcinoma, comparing said concentration C of autoantibodies to a threshold value y; wherein for C>y an increasing difference between said concentration C and said second threshold value y corresponds to a decreasing of survival free from progression and/or overall survival expectancy of said patient and for C<*y* an increasing difference between said concentration C and said threshold value y corresponds to an increasing of survival free from progression and/or overall survival expectancy of said patient.
wherein said determining in step a) is carried out by ELISA assay with detection of said antibodies with a peptide having a sequence selected from SEQ ID NO 1 or SEQ ID NO 3 or SEQ ID NO 4.

A further object of the invention is an *in vitro* method for the prognosis of survival free from progression and/or of overall survival expectancy in a patient affected by ovarian carcinoma, comprising the following steps:
a) determining the concentration C of anti-TUBB3 autoantibodies in a serum sample of said patient;
c) comparing said concentration C of autoantibodies to a threshold value y; wherein for C>y an increasing difference between said concentration C and said second threshold value y corresponds to a decreasing of survival free from progression and/or overall survival expectancy of said patient and for C<y an increasing difference between said concentration C and said threshold value y corresponds to an increasing of survival free from progression and/or overall survival expectancy of said patient.
wherein said determining in step a) is carried out by ELISA assay with detection of said antibodies with a peptide having a sequence selected from SEQ ID NO 1 or SEQ ID NO 3 or SEQ ID NO 4.

It's herein disclosed, as useful to understand the present invention, a peptide having a sequence corresponding to that of the last from 16 to 45 C-terminal amino acids of the human TUBB3 protein as described in the literature.

In other words, taking as an example isoform 1 of human TUBB3 having SEQ ID NO 2 (but also any one of the variants indicated in the above glossary, always having a 450-aa length), the peptide disclosed herein can have a sequence corresponding to the amino acids in position from 406 to 450, from 407 to 450, from 408 to 450, from 409 to 450, from 410 to 450, from 411 to 450, from 412 to 450, from 413 to 450, from 414 to 450, from 415 to 450, from 416 to 450, from 417 to 450, from 418 to 450, from 419 to 450, from 420 to 450, from 421 to 450, from 422 to 450, from 423 to 450, from 424 to 450, from 425 to 450, from 426 to 450, from 427 to 450, from 428 to 450, from 429 to 450, from 430 to 450, from 431 to 450, from 432 to 450, from 433 to 450, from 434 to 450, from 435 to 450 of human TUBB3, e.g. as represented in SEQ ID NO 2, or, in other words, the last 16, 17, 18, 19, 20, 21, 22, 23, 24, 25, 26, 27, 28, 29, 30, 31, 32, 33, 34, 35, 36, 37, 38, 39, 40, 42, 43, 43, 44 or 45 C-terminal amino acids of the human TUBB3 protein as described in the literature.

The peptide disclosed herein has a sequence corresponding to that of the last from 22 to 32 C-terminal amino acids of the human TUBB3 protein as described in the literature. In other words, taking as an example the isoform 1 of human TUBB3 having SEQ ID NO 2 (but also any one of the variants indicated in the glossary above, always having a 450-aa length), the peptide disclosed herein can have a sequence corresponding to the amino acids in position from 419 to 450, from 420 to 450, from 421 to 450, from 422 to 450, from 423 to 450, from 424 to 450, from 425 to 450, from 426 to 450, from 427 to 450, from 428 to 450, from 429 to 450 of human TUBB3, e.g. as represented in SEQ ID NO 2, or, in other words the last 22, 23, 24, 25, 26, 27, 28, 29, 30, 31 or 32 C-terminal amino acids of the human TUBB3 protein as described in the literature.

The peptide disclosed herein for carrying out the methods of the invention has a sequence corresponding to the amino acids in position from 423 to 450, from 424 to 450, or from 425 to 450 of human TUBB3, e.g. as represented in SEQ ID NO 2, or, in other words, the last 26, 27, or 28 C-terminal amino acids of the human TUBB3 protein as described in the literature.

The peptide for carrying out the methods of the invention as defined in the appended set of claims has a sequence selected from SEQ ID NO 1, SEQ ID NO 3, or SEQ ID NO 4. In a particularly preferred embodiment, the peptide of the invention has SEQ ID NO 1. The threshold value x can be calculated by comparing the serum distribution curves of anti-TUBB3 autoantibodies of samples collected from patients with benign gynaecological pathologies and the serum distribution curves of anti-TUBB3 autoantibodies of samples collected from patients affected by ovarian carcinoma and using a ROC curve for defining said threshold value according to the desired specificity and sensitivity.

According to one embodiment, the threshold value between pathological and normal levels of anti-TUBB3 autoantibodies can be extrapolated from a ROC curve. In practice, said analysis compares distribution curves of the seric (serum) levels of anti-TUBB3 antibodies in subjects affected by benign pathologies-and in subjects affected by ovarian carcinoma, analyses the degree of overlapping of the two distribution curves, and enables to calculate a threshold level (cut-off) which minimizes false-positive and false-negative mistakes.

The threshold value obtained through ROC curve analysis can be established based on sensitivity and desired specificity and is able to discern, e.g., a set of subjects affected by benign gynaecological pathologies from a set of ovarian carcinoma-affected subjects, by analysing the area subtended by the ROC curve (Area Under Curve, AUC). This is equivalent to the probability that the result of the test carried out on an individual randomly extracted from the group of diseased subjects be higher than that of one randomly extracted from the group of subjects affected by benign pathologies.

For the purposes of the present invention, the concentration C of the anti-TUBB3 autoantibodies, as well as all the threshold values, x and y can be expressed in terms of concentrations/volume or weight, or also in terms of absorbance at a suitable wavelength, when detection means comprising a spectrophotometric reading of the results are used.

Apparently, any methodology directly or indirectly determining the concentrations of anti-TUBB3 autoantibodies in serum may be used to implement the methods of the present invention and for the carrying out the kits described hereinafter.

As mentioned above, therefore, the threshold value, as well as the concentration C of autoantibodies in the serum of analysed patients, can be expressed as absorbance values at a suitable wavelength (depending on the detection system used). Apparently, the same system of values will be used for the determining of the concentration of anti-TUBB3 autoantibodies in the serum and for the determining of the threshold values x, and/or y.

Apparently, given a certain absorbance at a certain wavelength with a known protocol and detection system, it is possible to extrapolate from obtained absorbance values the corresponding concentrations of TUBB3 autoantibodies.

According to one embodiment of the present invention, the concentration C and the threshold value can therefore be expressed in terms of absorbance at a wavelength of between 500 and 400 nm, e.g. 500 nm, 490 nm, 480 nm, 470 nm, 460 nm, 450 nm, 440 nm, 430 nm, 420 nm, 410 nm, 400 nm. According to a specific embodiment, e.g. when using peroxidase in the detection system, said absorbance will be an absorbance at the wavelength of 450 nanometres, A₄₅₀, and said threshold value x could be a threshold value of between 0.09 and 0.05 as absorbance at a 450nm wavelength (A₄₅₀). The technician in the field, by knowing the protocol used in detail, can easily extrapolate the corresponding threshold values in terms of antibody concentrations, therefore the object applications also relates to the embodiment wherein said threshold values are expressed in terms of antibody concentration. The values report in terms of absorbance have therefore precise equivalents in terms of antibody concentrations. According to one embodiment of the invention, therefore, the threshold value x, is A₄₅₀ of between 0.09 and 0.05.

According to a further embodiment, said threshold value x corresponds to A₄₅₀, equal to 0.075 as cut-off between levels of anti-TUBB3 antibodies in subjects affected by benign pathology (A₄₅₀ ≤0.075) and in subjects affected by ovarian carcinoma (A₄₅₀ >0.075), with a calculated 95% sensitivity and 98% specificity percentage (AUC value equal to 0.99). As mentioned above, all these values are quickly translatable by the technician in the field in terms of antibody concentrations, based on the data provided in the experimental section of the application.

According to the invention, the threshold value y is any value of anti-TUBB3 autoantibodies serum concentration in a pool of samples collected from patients affected by ovarian carcinoma.

According to a specific embodiment, said threshold value y is equal to or higher than said threshold value x.

According to a further embodiment, said threshold value y is given by the median of the values of the anti-TUBB3 autoantibodies serum concentration in a pool of samples collected from patients affected by ovarian carcinoma.

According to the present invention, a "high" concentration of anti-TUBB3 autoantibodies corresponds to a concentration above the median, whereas a "low" concentration of anti-TUBB3 autoantibodies corresponds to a concentration below or equal to the median.

In this case as well, the threshold value y could be expressed as absorbance value at a suitable wavelength (depending on the detection system used).

Hence, according to one embodiment of the present invention, the concentration C and the different threshold values can therefore be expressed in terms of absorbance at a wavelength of between 500 and 400 nm, e.g.: 500 nm, 490 nm, 480 nm, 470 nm, 460 nm, 450 nm, 440 nm, 430 nm, 420 nm, 410 nm, 400 nm. According to a specific embodiment, e.g. when using peroxidase in the detection system, said absorbance will be an absorbance at the wavelength of 450 nanometres, A₄₅₀.

The above-described method, comprising also step c), enables the medical team to predict the survival free from progression and/or the overall survival of examined patients.

This method can therefore be introduced in a therapeutic method followed by a therapeutic step d) in which the patient is treated in a personalized manner based on the calculated survival free from progression and/or overall survival. Therefore, for patients with a reduced survival free from progression and/or overall survival expectancy, the medical team will more promptly carry out a surgical removal of ovarian carcinoma when possible, adjust the therapeutic regimen by modulating it depending on the known higher aggressiveness of the disease, carry out a stricter serial control over time in order to early identify disease progression, and an adequate counselling and psycho-oncological support for the patient and her family.

According to a specific embodiment, the above method, comprising steps a) b) and c), can also be carried out on patients for whom a diagnosis of ovarian carcinoma has already been made, and could therefore be carried out in a form limited to the sole steps a) and c) of prognosis of survival free from progression and/or of overall survival expectancy in a patient affected by ovarian carcinoma comprising the steps as described above.

The advantages deriving from the methods of the invention are immediately evident.

According to the invention, the determining in step a) of the above-reported methods is carried out by ELISA assay.

This technique, besides being quick and easily repeatable, also has the advantage that the ELISA assay ensures a semiquantitative photometric dosage of the analyte, obtained with continuous values of absorbance, and provides an objective result in comparison with the subjective (operator-dependent) and semiquantitative assessment of immunohistochemistry methodologies used in the state of the art of diagnostics.

According to this embodiment, the threshold value x can be expressed in terms of serum concentration of the autoantibody, or in terms of absorbance and can be, e.g., A₄₅₀ of between 0.09 and 0.05.

According to a specific embodiment of the above, said threshold value x can be equal to A₄₅₀ 0.075 and represent the cut-off between levels of anti-TUBB3 antibodies in subjects affected by a benign pathology (A₄₅₀ ≤0.075) and in subjects affected by ovarian cancer >0.0750,), with a calculated sensitivity percentage of 95% and specificity percentage of 98% (AUC value equal to 0.99).

It's also herein disclosed, as useful to understand the present invention,a peptide having a sequence corresponding to that of the last from 16 to 45 C-terminal amino acids of the human TUBB3 protein as described in the literature.

In other words, taking as an example the isoform 1 of human TUBB3 having SEQ ID NO 2 (but also any one of the variants indicated in the glossary above, always having a 450-aa length), said peptide can have a sequence corresponding to the amino acids in position from 406 to 450, from 407 to 450, from 408 to 450, from 409 to 450, from 410 to 450, from 411 to 450, from 412 to 450, from 413 to 450, from 414 to 450, from 415 to 450, from 416 to 450, from 417 to 450, from 418 to 450, from 419 to 450, from 420 to 450, from 421 to 450, from 422 to 450, from 423 to 450, from 424 to 450, from 425 to 450, from 426 to 450, from 427 to 450, from 428 to 450, from 429 to 450, from 430 to 450, from 431 to 450, from 432 to 450, from 433 to 450, from 434 to 450, from 435 to 450 of human TUBB3, e.g. as represented in SEQ ID NO 2, or, in other words the last 16, 17, 18, 19, 20, 21, 22, 23, 24, 25, 26, 27, 28, 29, 30, 31, 32, 33, 34, 35, 36, 37, 38, 39, 40, 42, 43, 43, 44 or 45 C-terminal amino acids of the human TUBB3 protein as described in the literature.

The peptide disclosed herein has a sequence corresponding to that of the last from 22 to 32 C-terminal amino acids of the human TUBB3 protein as described in the literature. In other words, taking as an example the isoform 1 of human TUBB3 having SEQ ID NO 2 (but also of the variants indicated in the glossary above, always having a 450-aa length), the peptide disclosed herein can have a sequence corresponding to the amino acids in position from 419 to 450, from 420 to 450, from 421 to 450, from 422 to 450, from 423 to 450, from 424 to 450, from 425 to 450, from 426 to 450, from 427 to 450, from 428 to 450, from 429 to 450 of human TUBB3, e.g. as represented in SEQ ID NO 2, or, in other words, the last 22, 23, 24, 25, 26, 27, 28, 29, 30, 31 or 32 C-terminal amino acids of the human TUBB3 protein as described in the literature.

The peptide disclosed herein has a sequence corresponding to the amino acids in position from 423 to 450, from 424 to 450 or from 425 to 450 of human TUBB3, e.g. as represented in SEQ ID NO 2, or, in other words the last 26, 27, or 28 C-terminal amino acids of the human TUBB3 protein as described in the literature.

Another object of the invention is a peptide having a sequence selected from SEQ ID NO 1 or SEQ ID NO 3.

In a particularly preferred embodiment, the peptide of the invention has SEQ ID NO 1.

According to the invention, the peptide can be used as such, or bound to a molecule allowing a better anchoring thereof for the coating of plates for dosage of auto-anti-TUBB3 antibodies by ELISA methodology. In any one of the embodiments described or claimed herein, any method known in the literature and commonly used in ELISA assays to foster a peptide adhesion to the matrix of the plates could be selected by the expert in the field to treat the peptide. Moreover, also the plate could be selected and/or treated in order to improve the anchoring system, and more generally the assay performance. The peptide of the invention in any one of the embodiments described or claimed herein could e.g. be biotinylated and analysed with suitable plates, such as, e.g., streptavidin- or neutravidin-coated plates.

Object of the invention is also the use of the peptide as defined in the appended claims for the carrying out of the methods of the invention in any one of the embodiments claimed herein.

A further object of the invention is a kit for ELISA assay (or ELISA kit) for the diagnosis of ovarian carcinoma and/or for the prognosis of survival free from progression and/or of overall survival expectancy of patients affected by ovarian carcinoma comprising one or more aliquots of reagents for the detection of anti-TUBB3 antibodies in serum samples and one or more control internal references for the quantification of these autoantibodies, wherein said reagents for the detection of anti-TUBB3 autoantibodies comprise a peptide as defined in the appended claims.

In other words, object of the invention is an ELISA kit for the diagnosis of ovarian carcinoma and/or for the prognosis of survival free from progression and/or of overall survival expectancy of patients affected by ovarian carcinoma, comprising one or more aliquots of reagents for the detection of anti-TUBB3 antibodies in serum samples and one or more control internal references for the quantification of these autoantibodies, wherein said reagents for the detection of anti-TUBB3 autoantibodies comprise a peptide as defined in the appended claims. The suggested assay being an indirect ELISA, the kit could further comprise optional reagents, like, e.g.: 1) enzyme bound to the secondary antibody (HRP, AP, galactosidase, etc), or even 2) the substrate used (for colorimetric measuring, in chemiluminescence or chemifluorescence).

Since the threshold values x or y might vary slightly depending on the cohort of patients analysed, and to make the assay immediately readable, the kit diagnostic/prognostic/predictive kit of the present invention is not merely a kit enabling the detection of anti-TUBB3 autoantibodies, but is a kit enabling to immediately establish the amount of anti-TUBB3 autoantibodies present in serum.

Thus, the kit enables an immediate reading of the results regardless of assigned threshold values that, as mentioned, can also vary simply according to the desired sensitivity and specificity.

Specifically, the ELISA kit according to the present invention comprises at least two or more control internal references for the quantification of said anti-TUBB3 antibodies in the form of wells in which known, increasing concentrations of humanized anti-TUBB3 antibody are dispensed.

The bonding between peptide, as defined in any one of the above-described embodiments, in each well and the AUTOTUBB3 present in the serum, after addition of secondary antibody and chromogen, determines the A_{nm values} at a spectrophotometric reading (e.g., the A₄₅₀ range expected in the studied population of patients affected by ovarian carcinoma as reported in Table 2 is between 0.05 and 0.44).

In one embodiment of the invention, the internal control references are composed of rows of wells in which known, increasing concentrations of humanized anti-TUBB3 antibody are dispensed, so to provide detailed information on the concentration of anti-TUBB3 autoantibodies for each serum sample analysed (e.g., in rows or in columns). It's also herein disclosed, as useful to understand the present invention, the use of a kit comprising reagents for the quantitative detection of anti-TU BB3 autoantibodies in serum or of reagents for the quantitative detection of anti-TUBB3 autoantibodies in serum in one of the embodiments described herein for carrying out any one of the above-described methods object of the present invention.

According to the description and the following embodiments, the term "low anti-TUBB3 autoantibodies" means a concentration of autoantibodies (expressed in terms of concentration or also in terms of absorbance) below the median or equal to the median of serum concentration of anti-TUBB3 autoantibodies measured in a sample of patients affected by ovarian carcinoma of reference.

According to the description and the following examples, the term "high anti-TUBB3 autoantibodies" means a concentration of autoantibodies (expressed in terms of concentration or also in terms of absorbance) above the median of serum concentration of anti-TUBB3 autoantibodies measured in a sample of patients affected by ovarian carcinoma of reference.

The term "expression levels of anti-TUBB3 autoantibodies" can also correspond to the term "concentration of anti-TUBB3 autoantibodies" as such values are clearly correlated with each other.

The Authors analysed sera obtained from 99 informed patients with epithelial ovarian tumour and from 80 informed patients with benign gynaecological pathologies. Sera were obtained at admission, prior to any therapeutic intervention on patients. Samples were analysed in triplicate by ELISA technique related to the biotinylated peptide/streptavidin-coated plate system. Diagnostic accuracy was evaluated through analysis of the area subtended by the ROC (Receiver Operating Characteristics) curve (Area Under Curve, AUC). Under the experimental conditions used, AUC proved equal to 0.99, denoting a highly accurate assay. Moreover, the ROC curve enabled the identification of the optimal threshold value (the so-called best cut-off), i.e., the value of the assay which maximizes the difference between true positives and false positives. Under the experimental conditions used, the best threshold value proved equal to 0.075 OD (OD: optical density), a value associated to a 95% sensitivity and a 97.5% specificity. The AUC, Cut-off, Sensitivity and Specificity values obtained using as antigen the entire TUBB3 protein (as per preceding patent: Patent application N° 102016000037019; Application Number: PCT/IB2017/052048) and the peptide object of the present invention are reported hereinafter.

### Protein as antigen

| **Area under the ROC curve** | |
|---|---|
| Area | 0.9341 |
| Standard Error | 0.02684 |
| 95% confidence interval | 0.8815 to 0.9867 |
| P value | < 0.0001 |
| Optimal cut-off | 0.15 |
| Specificity at 0.15 cut-off | 90.9 |
| Sensitivity at 0.15 cut-off | 91.8 |

### 27-aa biotinylated peptide as antigen

| **Area under the ROC curve** | |
|---|---|
| Area | 0.9939 |
| Standard Error | 0.003779 |
| 95% confidence interval | 0.9865 to 1.001 |
| P value | < 0.0001 |
| Optimal cut-off | 0.075 |
| Specificity at 0.15 cut-off | 98 |
| Sensitivity at 0.15 cut-off | 95 |

Streptavidin-coated plates were used with the biotinylated peptide in order to better anchor the peptide to the plate.

The examples below aim at providing experimental data obtained and possible embodiments of the invention, without however being binding. The expert in the field could refer to the detailed description of the invention in order to carry out simple modifications of what exemplified below, successfully carrying out the claimed method. On-plate immobilization of the peptide could be carried out according any method known in the literature and commonly used to foster adhesion of a peptide to an ELISA assay plate.

### EXAMPLES

The invention was developed through 4 phases: **1)** Blood sample collection from ovarian cancer patients and from patients affected by benign gynaecological pathologies; the patients had given their free and informed consent for collection and use of the biological material collected in the samples; **2)** Identification and set-up of optimal analysis conditions. **3)** Sample analysis and analytical validation of the diagnostic assay. **4)** Statistical processing of obtained results.

### Phase 1. Retrospective collection of serum samples to be analysed

For research purposes, 99 patients with malignant epithelial ovarian neoplasia treated from February to December 2016 were identified. Patients with different ovarian tumour histotypes, as well as different stages of the disease, were included in the collection. Patients' features are reported in Table 2. Moreover, a cohort of 80 women with benign gynaecological pathologies (such as ovarian cysts, uterine myomas, etc.) treated in the same time period was selected as reference population. Serum was obtained at patient admission, prior to any therapeutic intervention.

**Table 2. Clinicopathological features of ovarian tumour patients**

| **Feature** | **# of patients (%)** |
|---|---|
| **All cases** | 99 |
| **Median age, years (range)** | 56 (26-80) |
| **Tumour isotype** | |
| Serous | 80 (80.8) |
| Endometrioid | 4 (4.0) |
| Clear-cell | 8 (8.1) |
| Mucinous | 7 (7.1) |
| **FIGO Stage** | |
| I-II | 25 (25.3) |
| III-IV | 74 (74.7) |
| **Tumour grade** | |
| G1 | 7 (7.1) |
| G2-G3 | 92 (92.9) |

### Phase 2. Identification and set-up of optimal analysis conditions

Despite the analytical validity of the preceding results obtained with the use of the entire recombinant protein TUBB3 for serum determination of anti-TUBB3 autoantibodies (IT

Patent Application N° 102016000037019; Application Number: PCT/IB2017/052048), the complexity and the high costs related to recombinant protein production and purification were a big restriction for the development of a kit of easy use in clinical practice. The Authors therefore evaluated, as an alternative to the whole protein, a peptide corresponding to the specific portion of the TUBB3 protein (15 aa, in C-terminal). However, the results obtained from sample analysis under these experimental conditions did not determine acceptable assay sensitivity and specificity values. Synthesis and purification of a longer peptide (total 27 aa, QYQDATAEEEGEMYEDDEEESEAQGPK) corresponding to the C-terminal portion of TUBB3 protein, and subsequent biotinylation, remarkably improved assay performance, thanks to the use of a streptavidin-coated plate. Biotin is a small molecule that can be conjugated to very many proteins without losing or altering their activity, and each protein can bind several biotin molecules. Streptavidin is a tetramer protein with a very high affinity for biotin. Therefore, streptavidin-coated surfaces represent a powerful and universal tool for binding every type of biotinylated molecule (proteins, peptides, polysaccharides, oligonucleotides, DNA fragments, etc.) and since each streptavidin unit binds a biotin molecule, the result is a remarkable increase of assay sensitivity.

Different experimental conditions were tested by using plates with different streptavidin amounts and different dilutions of biotinylated peptide. The streptavidin-coated plate (binding ability 125 pmol -biotin/well) jointly to a 1:1000 dilution of the peptide yielded the most satisfactory results under the Authors' assay conditions. The "comparative test" with the ELISA methodology using the whole protein (related to the preceding patent) was carried out by dosing with the new method (biotinylated peptide-streptavidin-coated plate) the levels of anti-TUBB3 antibodies in 20 patients, previously analysed by using the whole protein, and calculating the correlation between OD (Optical Density) values for each patient, obtaining the results reported in Figure 1.

### Phase 3. Sample analysis

Upon validating the new method of biotinylated peptide/streptavidin-coated plate dosage, sample analysis was carried out in triplicate. As a control of the specific binding of anti-TUBB3 antibodies to the antigen, the serum of each patient was processed and analysed in a peptide-free well. The absorbance recorded in the latter was subtracted from the median of the peptide-containing samples.

### Phase 4. Statistical processing of obtained results

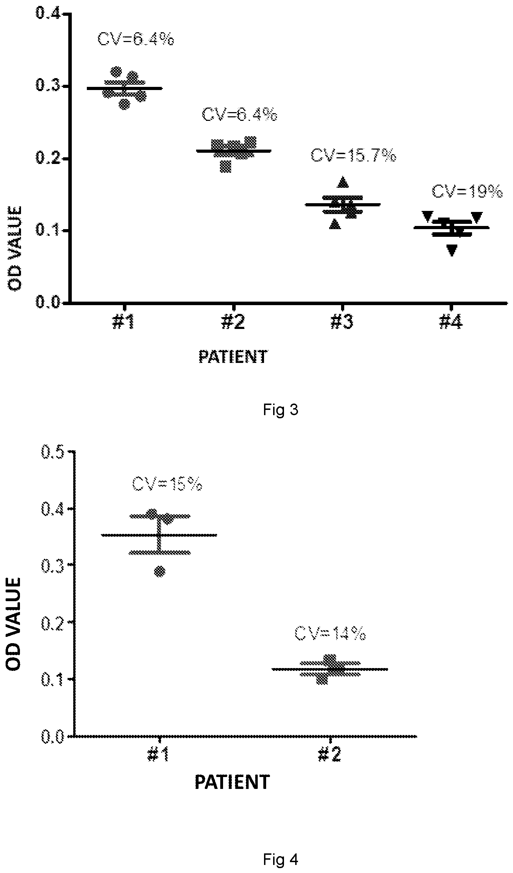

The diagnostic accuracy of the assay was evaluated through analysis of the area subtended by the ROC (Receiver Operating Characteristics) curve (Area Under Curve, AUC) **(****Figure 2****).** Under the experimental conditions used, AUC proved equal to 0.99, denoting a highly accurate assay. Moreover, the ROC curve enabled the identification of the optimal threshold value (the so-called best cut-off), i.e., the value of the assay which maximizes the difference between true positives and false positives. Under the experimental conditions used, the best threshold value proved equal to 0.075 OD (OD: optical density), a value associated to a 95% sensitivity and a 97.5% specificity. Analytical validation included determining intra- and inter-assay reproducibility **(****Figures 3 and 4****-6.** Intra-assay variation coefficients were calculated by testing at least 5 replicates of serum samples with decreasing concentrations of anti-TUBB3 autoantibodies. The variation coefficient (CV%) was determined to be of 6.4-19%, therefore deemed acceptable. Inter-assay variability was instead calculated by assaying two serum samples with high and low values of anti-TUBB3 antibodies in triplicate on 3 different plates. The variation coefficient (CV%) was of 15% and of 14% for samples with high and with low levels of anti-TUBB3 antibodies, respectively.

### 5. Serum collection:

5 ml of blood were collected from each patient in an anticoagulant-free test tube suitable for serum. Centrifuged at 4°C at 2,000 rcf and aliquoted into 0.5ml test tubes. Frozen at -80°C.

### 6. Preparation of kit components:

### Preparation of peptide for ELISA assay:

- A 1000X solution of biotinylated peptide TUBB3 (27 aa; PM: 3334; Weight: 5.4 mg) in filtered 1X PBS is prepared (final concentration 1mM). The 5-µl aliquots of peptide are stored into 1.5 ml test tubes at -80°C.
- On analysis day, a 5-µl aliquot of peptide is thawed in melting ice and then diluted 1X PBS 1:1000, to a 1µM concentration.

### Wash solution (Buffer PBS + 1% Tween 20):

- 5 PBS tablets (Fisher Scientific Bio-Cell) are dissolved in 1 I distilled water. For each 50 ml of 1X PBS solution, 50 µl of Tween 20 are added and the test tubes carefully stirred.

### Sera preparation:

- A serum aliquot for each patient is thawed. Thawing must be complete in order to make the solution homogeneous. The test tubes are carefully stirred.
- 1 microlitre of serum is collected and diluted into 100 microlitres of Buffer PBS + 1% Tween 20 + 1% gelatine. Serum dilution is very important in order to decrease the matrix effect and to bring the analyte concentration into the dosage linearity range.

### Secondary antibody preparation:

- 1 microlitre of the Goat anti-Human IgG-HRP secondary antibody (cod: F2708-MA80D, Beckman Coulter) solution is collected and diluted in 2 ml of 1X PBS. The best result is obtained at this dilution.

### 7. Dosage procedure:

- a 5-µl aliquot of peptide is thawed on melting ice.
- The peptide is diluted 1:1000 with 1X PBS, to bring it to a 1µM concentration.
- By using a 5-ml combitips, in the streptavidin-coated plate (Pierce Thermo Scientific Waltham, MA USA) wells 100 µl peptide are added for coating, incubating overnight at 4°C, under stirring (avoiding wells to be used as blank).
- On the next morning, the solution for saturating aspecific sites (1X PBS + 1% Tween and 1% Gelatine) is prepared in a 50-ml test tube. The test tube is placed into a 37°C thermostated bath to completely dissolve the gelatine.
- Excess Peptide is eliminated with the 12-channel multichannel pipette and washed twice with buffer (1X PBS + 1% Tween) using 5-ml combitips (100 µl).
- For each patient to be analysed, one aliquot of serum is thawed.
- Using the 5-ml combitips, 100µl of PBS/1%Tween/1%Gelatine are added into each well and left to incubate at 37°C for 1h.
- Pre-thawed sera, after stirring for at least 10 seconds, are diluted 1:100 with PBS/1% Tween/1% Gelatine and stirred again. The serum of each patient is analysed in triplicate.
- Excess PBS/1% Tween/ 1% Gelatine is eliminated with the 12-channel multichannel pipette, and the diluted patients' sera are directly added, letting incubate for 1h at 37°C.
- Excess serum is eliminated with the 12-channel multichannel pipette, and washing is carried out with buffer, using the 5-ml combitips.
- the Goat anti-Human IgG HRP-conjugated secondary antibody (already pre-diluted 1:2000 with 1X PBS) is taken and stirred for 10 seconds. 100 µl of the antibody is added into each well of the plate, letting incubate for 1h at 37°C.
- Goat anti-Human IgG HRP-conjugated secondary antibody is eliminated with the 12-channel multichannel pipette and washed with the wash buffer.
- Under a chemical hood, aided by 2.5 ml combitips, TMB chromogen is added into each well (100 µl) and left to incubate for about 1 minute.
- Reaction is blocked by adding 100 µl of Stop Solution (0.5M H₂SO₄).
- The plate is read at the spectrophotometer (EnSpire, Perkin Elmer) with a 450 nm filter.
- For each patient, blank (serum without peptide) value is subtracted from the median of the OD values of the three triplicates.

### 8. Patients:

A total of 99 patients with stage I-IV ovarian carcinoma, age range 26-80 years, was studied for the identification of serum concentration of IgG anti-TUBB3 autoantibodies with ELISA biotinylated peptide/streptavidin-coated plate methodology. The serum of ovarian carcinoma patients whose features are summarized in **Table 2** was collected at admittance prior to any therapeutic treatment. A population of 80 patients with benign gynaecological pathologies was used as control. All serum samples were stored at -80°C before being processed and studied.

### 9. ELISA (enzyme-linked immunosorbent assay):

For the purposes of the assay, the 27-aa peptide having SEQ ID NO 1 (sequences are indicated NH₂---COOH) was selected, corresponding to the C-terminal portion of the TUBB3 protein. The peptide was then biotinylated on the N-Terminal (peptide synthesis and biotinylation carried out by Primm s.r.l. Company, Italy). The use of streptavidin-coated plates in association with the biotinylated peptide allows to significantly strengthen the bonding chemistry, thereby obtaining a better performance of the assay. The first step consists in carrying out a "coating" of the bottom of a streptavidin-coated plate (Pierce Streptavidin high binding- binding capacity: 125 pmol D-biotin/well) with the biotinylated peptide, by incubating overnight at 4°C. After having removed the "coating" solution and washed the plate with wash buffer, the plate is incubated with a blocking solution for 1h at 37°C. Then, without washing the plate, the pre-thawed serum, diluted 1:100, is dispensed, incubating for 1h at 37°C. After a washing with buffer, the anti-human IgG HRP-conjugated secondary antibody is added for 1h at 37°C. After a further washing, the chromogen is added at room temperature to highlight the reaction. The reaction is blocked, and absorption at 450 nm is read using a plate reader. Each sample was analysed in triplicate. As control of the specific bonding of anti-TUBB3 antibodies to the antigen, the serum of each patient is processed and analysed in a peptide-free well. The absorbance recorded in the latter is subtracted from that detected in wells with peptide.

### 10. Statistical analysis.

As the ROC curve measures the accordance between the test (assay) of interest and the presence/absence of a specific disease, it represents the method of choice to validate a diagnostic test (assay). The ROC curve also enables to identify the optimal threshold value (the so-called *best cut-off*)*,* i.e. the test value maximizing the difference between true positives (that is, the ratio of individuals exhibiting an altered value of the test among all those actually affected by the disease, i.e., test sensitivity) and false positives (that is, the ratio of individuals who, though exhibiting an altered value of the test, are not affected by the disease of interest, i.e., test specificity).

## Claims

1. An *in vitro* method for the diagnosis of ovarian carcinoma in a patient, comprising the following steps:
a) determining the concentration C of anti-TUBB3 autoantibodies in a serum sample of said patient;
b) comparing said concentration C of autoantibodies to a threshold value x, wherein a concentration of said autoantibodies higher than said threshold value identifies the presence of ovarian carcinoma in said patient;
wherein said determining in step a) is carried out by ELISA assay with detection of said antibodies by a peptide consisting of a sequence selected from SEQ ID NO 1 or SEQ ID NO 3 or SEQ ID NO 4.

2. An *in vitro* method for the diagnosis of ovarian carcinoma and the prognosis of survival free from progression and/or of overall survival expectancy in case of presence of said ovarian carcinoma in a patient, comprising the following steps:
a) determining the concentration C of anti-TUBB3 autoantibodies in a serum sample of said patient;
b) comparing said concentration C of autoantibodies to a threshold value x, wherein a concentration of said autoantibodies higher than said threshold value x identifies the presence of ovarian carcinoma in said patient; and
c) when said patient is positive to ovarian carcinoma, comparing said concentration C of autoantibodies to a threshold value y; wherein for C>y an increasing difference between said concentration C and said second threshold value y corresponds to a decreasing of survival free from progression and/or overall survival expectancy of said patient and for C<y an increasing difference between said concentration C and said threshold value y corresponds to an increasing of survival free from progression and/or overall survival expectancy of said patient;
wherein said determining in step a) is carried out by ELISA assay with detection of said antibodies with a peptide consisting of a sequence selected from SEQ ID NO 1 or SEQ ID NO 3 or SEQ ID NO 4.

3. An *in vitro* method for the prognosis of survival free from progression and/or of overall survival expectancy in a patient affected by ovarian carcinoma, comprising the following steps:
a) determining the concentration C of anti-TUBB3 autoantibodies in a serum sample of said patient;
c) comparing said concentration C of autoantibodies to a threshold value y; wherein for C>y an increasing difference between said concentration C and said second threshold value y corresponds to a decreasing of survival free from progression and/or overall survival expectancy of said patient and for C<y an increasing difference between said concentration C and said threshold value y corresponds to an increasing of survival free from progression and/or overall survival expectancy of said patient;
wherein said determining in step a) is carried out by ELISA assay with detection of said antibodies with a peptide consisting of a sequence selected from SEQ ID NO 1 or SEQ ID NO 3 or SEQ ID NO 4.

4. The method according to anyone of claims 1 to 3, wherein said threshold value x is calculated by comparing the serum distribution curves of anti-TUBB3 autoantibodies of samples collected from individuals affected by benign gynaecological pathologies and the serum distribution curves of anti-TUBB3 autoantibodies of samples collected from patients affected by ovarian carcinoma and using a ROC curve for defining said threshold value according to the desired specificity and sensitivity.

5. The method according to anyone of claims 1 to 4 wherein said serum concentration of said autoantibodies and said threshold values x, and/or y are expressed in terms of concentration/volume or concentration/weight.

6. The method according to anyone of claims 1 to 5, wherein said serum concentration of said autoantibodies and said threshold values x, and/or y are expressed in terms of absorbance at a suitable wavelength.

7. The method according to claim 6, wherein said wavelength is 450 nm.

8. The method according to claim 7, wherein said threshold value x is between 0.09 and 0.05 or wherein said threshold value x is 0.075.

9. The method according to anyone of claims 5 to 8, wherein said desired specificity is between 97.6% and 99.5% or is higher than or equal to 97.8% and/or wherein said desired sensitivity is between 91.6% and 97% or is higher than or equal to 94.5%.

10. The method according to anyone of claims 2 to 9, wherein said threshold value y is any value indicating a serum concentration of anti-TUBB3 autoantibodies in pools of samples collected from patients affected by ovarian carcinoma

11. The method according to claim 10, wherein said threshold value y is equal to or higher than said threshold value x.

12. The method according to claim 11, wherein said threshold value y is given by the median of the values of the anti-TUBB3 autoantibodies serum concentration in samples collected from patients affected by ovarian carcinoma.

13. The method according to anyone of claims 1 to 12, wherein said peptide is immobilized on a plate for ELISA assay.

14. The method according to claim 13, wherein said peptide is biotinylated.

15. A peptide consisting of a sequence selected from SEQ ID NO 1 or SEQ ID NO 3.

16. The peptide according to claim 15, wherein said peptide is immobilized on a plate for ELISA assay.

17. The peptide according to claim 16, wherein said peptide is biotinylated.

18. Use of the peptide as defined in anyone of claims 15 to 17 for carrying out the method according to anyone of claims 1 to 14.

19. An ELISA kit for the diagnosis of ovarian carcinoma and/or for the prognosis of survival free from progression and/or of overall survival expectancy of patients affected by ovarian carcinoma, comprising one or more aliquots of reagents for the detection of anti-TUBB3 antibodies in serum samples and one or more control internal references for the quantification of these autoantibodies, wherein said reagents for the detection of anti-TUBB3 autoantibodies comprise a peptide as defined in anyone of claims 15 to 17.

20. The kit according to claim 19, wherein said one or more control internal references for the quantification of said anti-TUBB3 antibodies are wells wherein known, increasing concentrations of humanized anti-TUBB3 antibody are distributed and/or, wherein said wells containing known, increasing concentrations of humanized of humanized anti-TUBB3 antibody, are positioned in the ELISA plate so to provide detailed information on the concentration of anti-TUBB3 autoantibodies for each serum sample analysed.

## Patentansprüche

1. In-vitro-Verfahren zur Diagnose eines Ovarialkarzinoms bei einer Patientin, umfassend die folgenden Schritte:
a) Bestimmung der Konzentration C von Anti-TUBB3-Autoantikörpern in einer Serumprobe der Patientin;
b) Vergleichen der Konzentration C der Autoantikörper mit einem Schwellenwert x, wobei eine Konzentration der Autoantikörper, die höher als der Schwellenwert ist, das Vorhandensein eines Ovarialkarzinoms bei der Patientin identifiziert;
wobei das Bestimmen in Schritt a) durch eine ELISA-Untersuchung mit Nachweis der Antikörper durch ein Peptid durchgeführt wird, das aus einer Sequenz besteht, die aus SEQ ID NO 1 oder SEQ ID NO 3 oder SEQ ID NO 4 ausgewählt ist.

2. In-vitro-Verfahren zur Diagnose eines Ovarialkarzinoms und zur Prognose des Überlebens ohne Progression und/oder der Gesamtüberlebenserwartung bei Vorliegen des Ovarialkarzinoms bei einer Patientin, umfassend die folgenden Schritte:
a) Bestimmen der Konzentration C von Anti-TUBB3-Autoantikörpern in einer Serumprobe der Patientin;
b) Vergleichen der Konzentration C der Autoantikörper mit einem Schwellenwert x, wobei eine Konzentration der Autoantikörper, die höher als der Schwellenwert x ist, das Vorhandensein eines Ovarialkarzinoms bei der Patientin identifiziert; und
c) wenn die Patientin positiv auf ein Ovarialkarzinom ist, Vergleichen der Konzentration C der Autoantikörper mit einem Schwellenwert y; wobei für C>y eine zunehmende Differenz zwischen der Konzentration C und dem zweiten Schwellenwert y einer Abnahme des Überlebens ohne Progression und/oder der Gesamtüberlebenserwartung der Patientin entspricht und für C<y eine zunehmende Differenz zwischen der Konzentration C und dem Schwellenwert y einer Zunahme des Überlebens ohne Progression und/oder der Gesamtüberlebenserwartung der Patientin entspricht;
wobei das Bestimmen in Schritt a) durch eine ELISA-Untersuchung mit Nachweis der Antikörper mit einem Peptid durchgeführt wird, das aus einer Sequenz besteht, die aus SEQ ID NO 1 oder SEQ ID NO 3 oder SEQ ID N04 ausgewählt ist.

3. In-vitro-Verfahren zur Prognose eines progressionsfreien Überlebens und/oder einer Gesamtüberlebenserwartung bei einer an einem Ovarialkarzinom erkrankten Patientin, umfassend die folgenden Schritte:
a) Bestimmen der Konzentration C von Anti-TUBB3-Autoantikörpern in einer Serumprobe der Patientin;
c) Vergleichen der Konzentration C von Autoantikörpern mit einem Schwellenwert y; wobei für C>y eine zunehmende Differenz zwischen der Konzentration C und dem zweiten Schwellenwert y einer Abnahme des progressionsfreien Überlebens und/oder der Gesamtüberlebenserwartung der Patientin entspricht und für C<y eine zunehmende Differenz zwischen der Konzentration C und dem Schwellenwert y einer Zunahme des progressionsfreien Überlebens und/oder der Gesamtüberlebenserwartung der Patientin entspricht;
wobei das Bestimmen in Schritt a) durch eine ELISA-Untersuchung mit Nachweis der Antikörper mit einem Peptid durchgeführt wird, das aus einer Sequenz besteht, die aus SEQ ID NO 1 oder SEQ ID NO 3 oder SEQ ID NO 4 ausgewählt ist.

4. Verfahren nach einem der Ansprüche 1 bis 3, wobei der Schwellenwert x durch Vergleichen der Serumverteilungskurven von Anti-TUBB3-Autoantikörpern von Proben, die von Individuen gesammelt wurden, die von gutartigen gynäkologischen Pathologien betroffen sind, und der Serumverteilungskurven von Anti-TUBB3-Autoantikörpern von Proben, die von Patientinnen gesammelt wurden, die von einem Ovarialkarzinom betroffen sind, und unter Verwendung einer ROC-Kurve zur Definition des Schwellenwertes gemäß der gewünschten Spezifität und Sensitivität berechnet wird.

5. Verfahren gemäß einem der Ansprüche 1 bis 4, wobei die Serumkonzentration der Autoantikörper und die Schwellenwerte x und/oder y in Bezug auf Konzentration/Volumen oder Konzentration/Gewicht ausgedrückt werden.

6. Verfahren gemäß einem der Ansprüche 1 bis 5, wobei die Serumkonzentration der Autoantikörper und die Schwellenwerte x und/oder y in Bezug auf Absorption bei einer geeigneten Wellenlänge ausgedrückt werden.

7. Verfahren gemäß Anspruch 6, wobei die Wellenlänge 450 nm beträgt.

8. Verfahren gemäß Anspruch 7, wobei der Schwellenwert x zwischen 0,09 und 0,05 liegt oder wobei der Schwellenwert x 0,075 ist.

9. Verfahren gemäß einem der Ansprüche 5 bis 8, wobei die gewünschte Spezifität zwischen 97,6 % und 99,5 % ist oder höher als oder gleich 97,8 % ist und/oder wobei die gewünschte Empfindlichkeit zwischen 91,6 % und 97 % ist oder höher als oder gleich 94,5 % ist.

10. Verfahren gemäß einem der Ansprüche 2 bis 9, wobei der Schwellenwert y ein beliebiger Wert ist, der eine Serumkonzentration von Anti-TUBB3-Autoantikörpern in Probenpools angibt, die von Patientinnen gesammelt wurden, die von Ovarialkarzinom betroffen sind.

11. Verfahren gemäß Anspruch 10, wobei der Schwellenwert y gleich oder höher ist als der Schwellenwert x.

12. Verfahren gemäß Anspruch 11, wobei der Schwellenwert y durch den Median der Werte der Anti-TUBB3-Autoantikörper-Serumkonzentration in Proben gegeben ist, die von Patientinnen gesammelt wurden, die von Ovarialkarzinom betroffen sind.

13. Verfahren gemäß einem der Ansprüche 1 bis 12, wobei das Peptid auf einer Platte für eine ELISA-Untersuchung immobilisiert ist.

14. Verfahren gemäß Anspruch 13, wobei das Peptid biotinyliert ist.

15. Peptid, bestehend aus einer Sequenz, ausgewählt aus SEQ ID NO 1 oder SEQ ID NO 3.

16. Peptid gemäß Anspruch 15, wobei das Peptid auf einer Platte für eine ELISA-Untersuchung immobilisiert ist.

17. Das Peptid gemäß Anspruch 16, wobei das Peptid biotinyliert ist.

18. Verwendung des Peptids, wie in einem der Ansprüche 15 bis 17 definiert, zur Durchführung des Verfahrens gemäß einem der Ansprüche 1 bis 14.

19. ELISA-Kit für die Diagnose eines Ovarialkarzinoms und/oder für die Prognose eines Überlebens frei von Progression und/oder einer Gesamtüberlebenserwartung von Patientinnen, die von einem Ovarialkarzinom betroffen sind, umfassend ein oder mehrere Aliquots von Reagenzien für den Nachweis von Anti-TUBB3-Antikörpern in Serumproben und eine oder mehrere interne Kontrollreferenzen für die Quantifizierung dieser Autoantikörper, wobei die Reagenzien für den Nachweis von Anti-TUBB3-Autoantikörpern ein Peptid umfassen, wie es in einem der Ansprüche 15 bis 17 definiert ist.

20. Kit gemäß Anspruch 19, wobei die eine oder mehrere interne Kontrollreferenzen für die Quantifizierung der Anti-TUBB3-Antikörper Vertiefungen sind, in denen bekannte, ansteigende Konzentrationen von humanisierten Anti-TUBB3-Antikörpern verteilt sind, und/oder wobei die Vertiefungen, die die bekannten, ansteigenden Konzentrationen von humanisierten Anti-TUBB3-Antikörpern enthalten, in der ELISA-Platte so angeordnet sind, dass sie detaillierte Informationen über die Konzentration von Anti-TUBB3-Autoantikörpern für jede analysierte Serumprobe bereitstellen.

## Revendications

1. Méthode *in vitro* de diagnostic d'un carcinome ovarien chez une patiente, comprenant les étapes suivantes consistant à :
a) déterminer la concentration C d'auto-anticorps anti-TUBB3 dans un échantillon de sérum de ladite patiente ;
b) comparer ladite concentration C d'auto-anticorps à une valeur seuil x, où une concentration desdits auto-anticorps supérieure à ladite valeur seuil identifie la présence d'un carcinome ovarien chez ladite patiente ;
dans laquelle ladite détermination à l'étape a) est réalisée par dosage ELISA avec détection desdits anticorps par un peptide se composant d'une séquence sélectionnée à partir de la SEQ ID NO : 1 ou SEQ ID NO : 3 ou SEQ ID NO : 4.

2. Méthode *in vitro* de diagnostic d'un carcinome ovarien et de pronostic de survie libre de toute progression et/ou d'espérance de survie globale en cas de présence dudit carcinome ovarien chez une patiente, comprenant les étapes suivantes consistant à :
a) déterminer la concentration C d'auto-anticorps anti-TUBB3 dans un échantillon de sérum de ladite patiente ;
b) comparer ladite concentration C d'auto-anticorps à une valeur seuil x, où une concentration desdits auto-anticorps supérieure à ladite valeur seuil x identifie la présence d'un carcinome ovarien chez ladite patiente ; et
c) lorsque ladite patiente est positive à un carcinome ovarien, comparer ladite concentration C d'auto-anticorps à une valeur seuil y, où, pour C > y, une différence croissante entre ladite concentration C et ladite seconde valeur seuil y correspond à une diminution de la survie libre de toute progression et/ou de l'espérance de survie globale de ladite patiente et, pour C < y, une différence croissante entre ladite concentration C et ladite seconde valeur seuil y correspond à une hausse de la survie libre de toute progression et/ou de l'espérance de survie globale de ladite patiente ;
dans laquelle ladite détermination à l'étape a) est réalisée par dosage ELISA avec détection desdits anticorps par un peptide se composant d'une séquence sélectionnée à partir de la SEQ ID NO 1 : ou SEQ ID NO : 3 ou SEQ ID NO : 4.

3. Méthode *in vitro* de pronostic de survie libre de toute progression et/ou d'espérance de survie globale chez une patiente atteinte d'un carcinome ovarien, comprenant les étapes suivantes consistant à :
a) déterminer la concentration C d'auto-anticorps anti-TUBB3 dans un échantillon de sérum de ladite patiente ;
c) comparer ladite concentration C d'auto-anticorps à une valeur seuil y, où, pour C > y, une différence croissante entre ladite concentration C et ladite seconde valeur seuil y correspond à une diminution de la survie libre de toute progression et/ou de l'espérance de survie globale de ladite patiente et, pour C < y, une différence croissante entre ladite concentration C et ladite seconde valeur seuil y correspond à une hausse de la survie libre de toute progression et/ou de l'espérance de survie globale de ladite patiente ;
dans laquelle ladite détermination à l'étape a) est réalisée par dosage ELISA avec détection desdits anticorps par un peptide se composant d'une séquence sélectionnée à partir de la SEQ ID NO : 1 ou SEQ ID NO : 3 ou SEQ ID NO : 4.

4. Méthode selon l'une quelconque des revendications 1 à 3, dans laquelle ladite valeur seuil x est calculée en comparant les courbes de distribution de sérum des auto-anticorps anti-TUBB3 d'échantillons collectés auprès de personnes atteintes de pathologies gynécologiques bénignes et les courbes de distribution de sérum des auto-anticorps anti-TUBB3 d'échantillons collectés auprès de patientes atteintes d'un carcinome ovarien et en utilisant une courbe ROC pour définir ladite valeur seuil en fonction de la spécificité et de la sensibilité voulues.

5. Méthode selon l'une quelconque des revendications 1 à 4, dans laquelle ladite concentration de sérum desdits auto-anticorps et lesdites valeurs seuils x et/ou y sont exprimées en termes de concentration/volume ou concentration/poids.

6. Méthode selon l'une quelconque des revendications 1 à 5, dans laquelle ladite concentration de sérum desdits auto-anticorps et lesdites valeurs seuils x et/ou y sont exprimées en termes d'absorbance à une longueur d'onde appropriée.

7. Méthode selon la revendication 6, dans laquelle ladite longueur d'onde est 450 nm.

8. Méthode selon la revendication 7, dans laquelle ladite valeur seuil x est comprise entre 0,09 et 0,05 ou dans laquelle ladite valeur seuil x est 0,075.

9. Méthode selon l'une quelconque des revendications 5 à 8, dans laquelle ladite spécificité voulue est comprise entre 97,6 % et 99,5 % ou est supérieure ou égale à 97,8 % et/ou dans laquelle ladite sensibilité voulue est comprise entre 91,6 % et 97 % ou est supérieure ou égale à 94,5 %.

10. Méthode selon l'une quelconque des revendications 2 à 9, dans laquelle ladite valeur seuil y est une valeur quelconque indiquant une concentration de sérum d'auto-anticorps anti-TUBB3 dans des groupes d'échantillons collectés auprès de patientes atteintes d'un carcinome ovarien.

11. Méthode selon la revendication 10, dans laquelle ladite valeur seuil y est égale ou supérieure à ladite valeur seuil x.

12. Méthode selon la revendication 11, dans laquelle ladite valeur seuil y est donnée par la moyenne des valeurs de la concentration de sérum d'auto-anticorps anti-TUBB3 dans des échantillons collectés auprès de patientes atteintes d'un carcinome ovarien.

13. Méthode selon l'une quelconque des revendications 1 à 12, dans laquelle ledit peptide est immobilisé sur une plaque pour le dosage ELISA.

14. Méthode selon la revendication 13, dans laquelle ledit peptide est biotinylé.

15. Peptide se composant d'une séquence sélectionnée parmi la SEQ ID NO : 1 ou la SEQ ID NO : 3.

16. Peptide selon la revendication 15, ledit peptide étant immobilisé sur une plaque pour le dosage ELISA.

17. Peptide selon la revendication 16, ledit peptide étant biotinylé.

18. Utilisation du peptide tel que défini selon l'une quelconque des revendications 15 à 17 pour la réalisation de la méthode selon l'une quelconque des revendications 1 à 14.

19. Kit ELISA de diagnostic d'un carcinome ovarien et/ou de pronostic de survie libre de toute progression et/ou d'espérance de survie globale de patientes atteintes d'un carcinome ovarien, comprenant une ou plusieurs aliquotes de réactifs pour la détection d'anticorps anti-TUBB3 dans des échantillons de sérum et une ou plusieurs références internes témoins pour la quantification desdits auto-anticorps, dans lequel lesdits réactifs pour la détection d'auto-anticorps anti-TUBB3 comprennent un peptide tel que défini selon l'une quelconque des revendications 15 à 17.

20. Kit selon la revendication 19, dans lequel lesdites une ou plusieurs références internes pour la quantification desdits anticorps anti-TUBB3 sont des puits dans lesquels des concentrations croissantes connues d'anticorps anti-TUBB3 humanisés sont distribuées et/ou, dans lequel lesdits puits contenant des concentrations croissantes connues d'anticorps anti-TUBB3 humanisés sont positionnés dans la plaque ELISA afin de fournir des informations détaillées concernant la concentration d'auto-anticorps anti-TUBB3 pour chaque échantillon de sérum analysé.
